# EUROPEAN PATENT APPLICATION

(11) **EP 4 049 721 A1**
(43) Date of publication of application: **31.08.2022**
(21) Application number: 19949783.5
(22) Date of filing: 08.11.2019
(51) Int. Cl.: A61N 5/06

(54) **STOMATITIS TREATMENT DEVICE**

(30) Priority: 22.10.2019 KR 20190131346
(71) Applicant: Welsmeditech Co., Ltd., Chungcheongnam-do 31035 (KR)
(72) Inventor: HWANG, Kyong Won, Seoul 01197 (KR); MOON, Pyoung Su, Cheonan-si, Chungcheongnam-do 31101 (KR)
(74) Representative: Gulde & Partner
(86) International application number: PCT/KR2019/015208
(87) International publication number: WO 2021/080072

(57) **Abstract**

The present invention relates to a stomatitis treatment device which is equipped with a light emitting means inside a housing to enable inflammations on the roof of the mouth and the upper side of the tongue to be treated, and to which a coupling module is attached to allow inflammations occurring on the lower side of the bottom of the mouth, etc. to be treated. The stomatitis treatment device comprises: the housing, which is inserted into the oral cavity and rests on the roof of the mouth and the upper side of the tongue; a main body including the housing and a connection part; a cell regeneration lamp which is provided inside the housing and outputs near infrared rays; an external device for supplying power to the cell regeneration lamp; and the coupling module which is coupled to the housing, rests on the lower side of the tongue and the bottom of the mouth, and includes a light carrier. The stomatitis treatment device allows the roof of the mouth, the tongue, and the bottom of the mouth to be simultaneously irradiated with light for treatment by attaching and detaching the coupling module to and from the housing having the cell regeneration lamp therein, and thus can be used by patients having severe stomatitis with multiple lesions.

## Description

### [Technical Field]

The present invention relates to a stomatitis treatment device, and more particularly, to a stomatitis treatment device configured not only to treat inflammations at a palate and a top side of a tongue using a light emission device provided inside a housing but also to treat inflammations at a bottom side of the tongue and a floor of a mouth by attaching a coupling module thereto.

### [Background Art]

Generally, stomatitis refers to overall inflammatory diseases occurring on a tongue, a palate, a floor of a mouth, buccal mucosa, and the like. Generally, when slight stomatitis occurs, medicine is directly applied to an inside of an oral cavity. When serious stomatitis occurs, medicine is directly applied to an inside of an oral cavity and medicine for internal use is taken at the same time.

However, when medicine is directly applied to an inside of an oral cavity, the medicine is quickly washed away by saliva, and a treatment effect is not continued. Accordingly, a therapeutic period increases. Also, there is a problem in which it is difficult to prescribe medicines for internal use for children, some patients, pregnant women, and the like.

To solve the above problems, an oral remedy device including a mouthpiece set including an upper mouthpiece with a first insertion hole in front thereof and a lower mouthpiece with a second insertion hole in front thereof and a light emission portion disposed between the upper mouthpiece and the lower mouthpiece and configured to emit light toward an inside of an oral cavity and in which an adequate distance from a part to be treated may be maintained and an oral curer may be stably supported in the oral cavity has been developed, which is disclosed in detail in Korean Patent Registration No. 10-1858657 (registered on May 10, 2018).

Also, Korean Patent Publication No. 10-2013-0057692 (published on June 3, 2013) discloses an oral remedy mouthpiece including a body inserted into an oral cavity, a biting portion extending from the body and bitten by at least one of lips and teeth of a patient, and configured to support the body against the oral cavity, a beam forming portion disposed inside the body and configured to generate a beam for treatment, and a beam extension portion disposed between the beam forming portion and an open area of the body and configured to uniformly extend the beam generated by the beam forming portion toward an inside of the oral cavity so as to have a structure including the body and the biting portion which is easily bitten by the patient and to uniformly emit the beam for treatment toward the inside of the oral cavity and an oral remedy device including the same.

Also, Korean Patent Registration No. 10-0777438 (registered on November 12, 2007) discloses an optical periodontal disease treatment device including a mouthpiece member including an insertion furrow to be inserted into a mouth and to surround teeth and a part of the gums, a light source array in which a plurality of light sources are arranged in the mouthpiece member to emit light toward the teeth and the gums, and a controller configured to control driving of the light sources.

However, in the case of related arts including the above documents, since a mouthpiece in which the light sources are inserted has a fixed size, there is still a problem of the related arts in which it is difficult for a variety of patients having oral cavities having different sizes such as children, adults, and the like to use the related arts.

Also, in the related arts including the above documents, it is difficult to emit light in the case of stomatitis formed on a palate, buccal mucosa, and the like in addition to teeth and gums.

Also, in the case of related arts including the above documents, it is difficult to prevent trouble occurring due to an oral secretion flowing into a device even in an electronic device to which a mouthpiece, in which a controller and a light source array are installed, is connected through a driving wire.

### [Disclosure]

### [Technical Problem]

The present invention is directed to providing a stomatitis treatment device in which a coupling module is detachably attached to a housing including a cell restoration lamp so as to emit light toward a palate, a tongue, a floor of a mouth, and buccal mucosa at the same time so that not only a serious stomatitis patient may be treated but also an oral secretion prevention hole, an oral secretion prevention portion is provided so as to prevent a failure of an electronic device caused by an oral secretion of a patient.

### [Technical Solution]

One aspect of the present invention provides a stomatitis treatment device including a housing inserted into an oral cavity and mounted on a palate and a top side of a tongue, a body including the housing and a connection portion, a cell restoration lamp provided inside the housing and configured to output a near-infrared ray, an external device configured to supply power to the cell restoration lamp, and a coupling module coupled to the housing, mounted on a bottom side of the tongue and a floor of a mouth, and including a light transfer body. Here, it is possible to output near-infrared rays to the palate, the top side of the tongue, the bottom side of the tongue, and the floor of the mouth at the same time.

The housing may include a first upper portion formed to be convex to be mounted on the palate and a first lower portion formed to be concave to be mounted on the top side of the tongue. Also, the first lower portion may include a plurality of coupling grooves formed at a distance of 5 mm to 10 mm from an apex of the tongue mounted thereon.

The light transfer body may be embedded in and protrude from the coupling module. Also, the coupling module may include a second upper portion formed to have a gradient decreased in a direction from a surface coupled to and coming in contact with the housing toward a frenum of the tongue to allow the bottom side of the tongue to be mounted thereon and a second lower portion having a flat bottom side to be mounted on the floor of the mouth.

The housing may further include a transfer lamp configured to output the near-infrared rays. Here, a protruding part of the light transfer body may be coupled to the coupling groove. An embedded part of the light transfer body may include a plurality of ends and transfer light emitted from the transfer lamp to the plurality of ends. A plurality of light-inducing grooves may be formed outside the coupling module and the housing.

The connection portion may include a terminal formed of a silicone material and provided to be connected to the external device, a plurality of separation prevention portions protruding from an outside of the terminal to increase a coupling force of the terminal connected to the external device, and an oral secretion prevention portion provided on one side of the terminal to prevent an oral secretion from flowing into the terminal.

The oral secretion prevention portion may be formed to be concave toward the terminal so that the oral secretion is collected therein.

### [Advantageous Effects]

According to the present invention, a stomatitis treatment device in which a coupling module is detachably attached to a housing including a cell restoration lamp thereinside so as to emit light for treatment toward a palate, a floor of a mouth, and buccal mucosa at the same time may be used by serious stomatitis patients having multiple lesions.

Also, according to the present invention, the stomatitis treatment device may include an oral secretion prevention portion formed on one side of a terminal so as to prevent an oral secretion of a patient who wears a housing from flowing into a body or an external device.

### [Description of Drawings]

FIG. 1 is an exploded perspective view of a stomatitis treatment device according to an embodiment of the present invention.
FIG. 2 is a side cross-sectional view according to the embodiment of the present invention.
FIG. 3 is an enlarged view of the side cross-sectional view according to the embodiment of the present invention.
FIG. 4 is an exemplary view of using the stomatitis treatment device according to the embodiment of the present invention.

### [Modes of the Invention]

Hereinafter, an exemplary embodiment of the present invention will be described in detail with reference to the attached drawings.

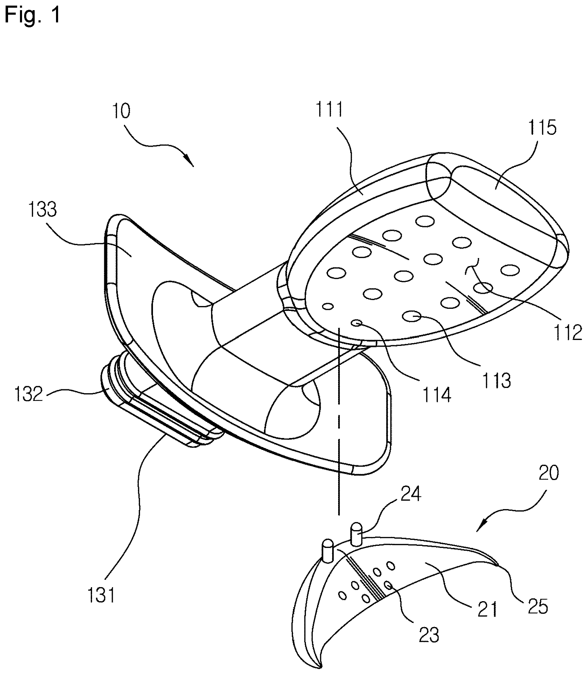

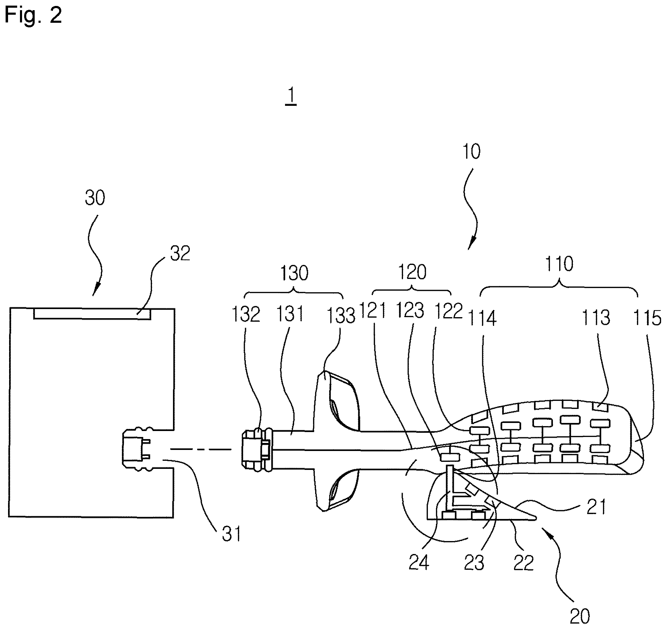

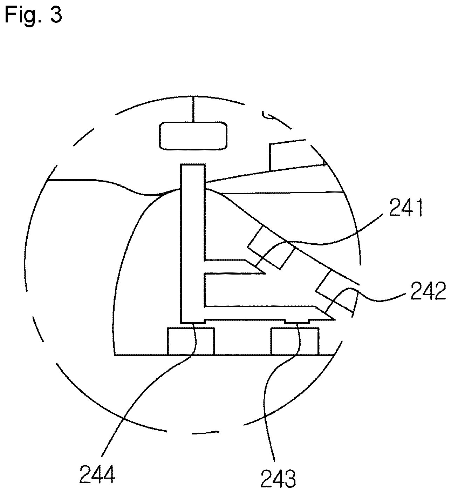

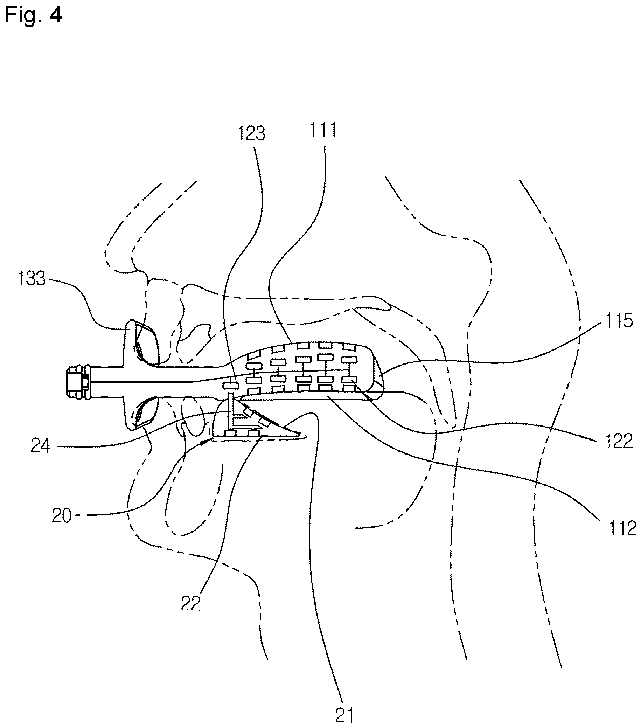

FIG. 1 is an exploded perspective view of a stomatitis treatment device according to an embodiment of the present invention, FIG. 2 is a side cross-sectional view according to an exemplary embodiment of the present invention, FIG. 3 is an enlarged view of the side cross-sectional view according to the embodiment of the present invention, and FIG. 4 is an exemplary view of using the stomatitis treatment device according to the embodiment of the present invention.

A stomatitis treatment device 1 according to the present invention may include a body 10, a coupling module 20 coupled to one side of the body 10, and an external device 30 coupled to one end of the body 10.

The body 10 includes a housing 110 which is inserted into an oral cavity, a substrate portion 120 formed inside the housing 110 to emit light, and a connection portion 130 configured to electrically connect the substrate portion 120 to an outside and has an exterior formed by molding with transparent silicone. A method of manufacturing the body 10 is not limited thereto and any manufacturing processes in which the substrate portion 120 is insertable and installable inside the body 10 may be applied.

As shown in FIGS. 2 to 4, the housing 110 includes a first upper portion 111 formed to have a shape insertable into an oral cavity of a patient and be convex upward to be mounted on a palate, a first lower portion 112 formed to be concave downward to be mounted on a top side of a tongue, a plurality of coupling grooves 114 formed at a distance of 5 mm to 10 mm from an apex of the tongue mounted on the first lower portion 112, and a uvula evasion portion 115 formed to have a concave center so as not to allow a uvula to come into contact therewith.

The substrate portion 120 includes a substrate 121, a cell restoration lamp 122, and a transfer lamp 123 as shown in FIG. 2.

The substrate 121 includes a flexible material like the housing 110, and the substrate 121 is embedded in an intermediate plane between the first upper portion 111 and the first lower portion 112.

Such cell restoration lamps 122 connected to the substrate 121 are built in the first upper portion 111 and the first lower portion 112.

The cell restoration lamp 122 is formed of a laser or a light emitting diode (LED) configured to output a near-infrared ray wavelength including 675 nm or 808 nm and which is emittable outward through the silicone of the housing 110. Since the cell restoration lamp 122 is able to output near-infrared rays, cell energy may be increased by activating an adenosine triphosphoric acid reaction of cells of an inflamed part. Due to the increased cell energy, cell restoration becomes vigorous so that it is possible to effectively treat stomatitis. Although the near-infrared lamp is used as the cell restoration lamp 122 in the above description, the present invention is not limited thereto, and any light sources which are effective in cell restoration such as an ultraviolet lamp or the like which is able to activate cell restoration by sterilizing harmful bacteria in the inflamed part through emission of ultraviolet rays may be applied.

Light output from the cell restoration lamp 122 may be emitted outward from the first upper portion 111 so as to treat inflammations occurring at the palate and buccal mucosa, may be emitted outward from the second lower portion 121 so as to treat inflammations occurring the top side of the tongue and buccal mucosa, and may be emitted outward from the uvula evasion portion 115 so as to treat inflammations occurring at the uvula and tonsils.

As shown in FIG. 2, the transfer lamp 123, like the cell restoration lamp, is embedded in the coupling groove 114.

As shown in FIG. 4, the coupling module 20 includes a light transfer body 24 embedded to protrude to be coupled to the coupling groove 114, a second upper portion 21 formed to have a lower gradient in a direction from a surface coming into contact with the housing toward a frenum of the tongue to allow a bottom side of the tongue to be mounted thereon, a second lower portion 22 having a flat bottom side to be mounted on a floor of a mouth, and a support portion 25 having both ends concave inward to be stably supported by the floor of the mouth and side surfaces of the lower gums and includes a transparent silicone material like the housing 110.

A protruding part of the light transfer body 24 may be forcibly inserted into the coupling groove 114 and the light transfer body 24 may be formed of a light-guiding member such as poly(methyl methacrylate) (PMMA) or include an optical fiber cable embedded therein so as to transmit light output from the transfer lamp 123.

As shown in FIGS. 2 and 3, a first end 241 and a second end 242 formed at an embedded part of the light transfer body 24 may transfer light output from the transfer lamp 123 to the second upper portion 21 so as to treat an inflammation occurring on the bottom side of the tongue.

A third end 243 and a fourth end 244 formed at the embedded part of the light transfer body 24 may transfer light output from the transfer lamp 123 to the second lower portion 22 so as to treat an inflammation occurring on the floor of the mouth.

Also, a plurality of light-inducing grooves 113 may be formed outside the housing 110 so as to emit light toward not only the palate and the top side of the tongue but also a rear of the gums, the buccal mucosa, and the tonsils.

A plurality of light-inducing grooves 23 may be formed outside the coupling module 20 so as to emit light toward not only the bottom side of the tongue and the floor of the mouth but also the buccal mucosa when coupled to the housing 110.

As described above, in the present invention, light may be emitted toward not only the palate, the top side of the tongue, the bottom side of the tongue, and the floor of the mouth but also the rear of gums, the buccal mucosa, and tonsils at the same time so as to treat a serious stomatitis patient having multiple lesions.

The connection portion 130 includes a terminal 131, a separation prevention portion 132, and an oral secretion prevention portion 133 as shown in FIG. 2.

The terminal 131 is electrically connected to the substrate 121. The terminal 131 may be connected to a terminal opening 31 of the external device 30, and power supply, light output intensity, an output time, wavelength, and the like of the cell restoration lamp 122 may be adjusted by manipulating a control panel 32.

A plurality of such separation prevention portions 132 protruding to increase a contact force with the terminal opening 31 are formed outside the terminal 131. A fastening force may be maintained by the separation prevention portions 132 in spite of a slight tremor of the patient who receives light treatment.

Since the patient who wears the housing 110 has a difficulty in smooth swallowing of the oral secretion, when the oral secretion flows outward from the housing and into the terminal 131 or the terminal opening 31, the stomatitis treatment device 1 may be broken due to a short circuit. To prevent this, the oral secretion prevention portion 133 having a rectangularly protruding shape is provided on one side of the terminal 131.

As shown in FIG. 1, the oral secretion prevention portion 133 has one concave surface so that lips may be mounted thereon or a certain amount of oral secretion may be temporarily collected.

## Claims

1. A stomatitis treatment device comprising:
a housing inserted into an oral cavity and mounted on a palate and a top side of a tongue;
a body comprising the housing and a connection portion;
a cell restoration lamp provided inside the housing and configured to output a near-infrared ray;
an external device configured to supply power to the cell restoration lamp; and
a coupling module coupled to the housing, mounted on a bottom side of the tongue and a floor of a mouth, and comprising a light transfer body,
wherein it is possible to output near-infrared rays to the palate, the top side of the tongue, the bottom side of the tongue, and the floor of the mouth at the same time.

2. The stomatitis treatment device of claim 1, wherein the housing comprises a first upper portion formed to be convex to be mounted on the palate and a first lower portion formed to be concave to be mounted on the top side of the tongue, and
wherein the first lower portion comprises a plurality of coupling grooves formed at a distance of 5 mm to 10 mm from an apex of the tongue mounted thereon.

3. The stomatitis treatment device of claim 2, wherein the light transfer body is embedded in and protrudes from the coupling module, and
wherein the coupling module comprises a second upper portion formed to have a gradient decreased in a direction from a surface coupled to and coming in contact with the housing toward a frenum of the tongue to allow the bottom side of the tongue to be mounted thereon and a second lower portion having a flat bottom side to be mounted on the floor of the mouth.

4. The stomatitis treatment device of claim 3, wherein the housing further comprises a transfer lamp configured to output the near-infrared rays,
wherein a protruding part of the light transfer body is coupled to the coupling groove,
wherein an embedded part of the light transfer body comprises a plurality of ends and transfers light emitted from the transfer lamp to the plurality of ends, and
wherein a plurality of light-inducing grooves are formed outside the coupling module and the housing.

5. The stomatitis treatment device of claim 1, wherein the connection portion comprises a terminal formed of a silicone material and provided to be connected to the external device, a plurality of separation prevention portions protruding from an outside of the terminal to increase a coupling force of the terminal connected to the external device, and an oral secretion prevention portion provided on one side of the terminal to prevent an oral secretion from flowing into the terminal.

6. The stomatitis treatment device of claim 5, wherein the oral secretion prevention portion is formed to be concave toward the terminal so that the oral secretion is collected therein.
